# EUROPÄISCHE PATENTSCHRIFT

(11) **EP 2 704 654 B1**
(45) Veröffentlichungstag und Bekanntmachung des Hinweises auf die Patenterteilung: **16.11.2022**
(21) Anmeldenummer: 12721412.0
(22) Anmeldetag: 04.05.2012
(51) Int. Cl.: A61B 18/04, A61L 2/14, A61B 18/00, H05H 1/46

(54) **VORRICHTUNG ZUR PLASMABEHANDLUNG VON OBERFLÄCHEN**
DEVICE FOR THE PLASMA TREATMENT OF SURFACES
DISPOSITIF DE TRAITEMENT DER SURFACES AU PLASMA

(30) Priorität: 05.05.2011 DE 102011100751; 25.05.2011 DE 102011102589
(43) Veröffentlichungstag der Anmeldung: 12.03.2014
(73) Patentinhaber: Max-Planck-Gesellschaft zur Förderung der Wissenschaften e.V., 80539 München (DE)
(72) Erfinder: MORFILL, Gregor, 81927 München (DE); LI, Yangfang, 85748 Garching (DE); ZIMMERMANN, Julia, 81925 München (DE); SHIMIZU, Tetsuji, 85748 Garching (DE)
(74) Vertreter: Kordel, Mattias
(86) Internationale Anmeldenummer: PCT/EP2012/001924
(87) Internationale Veröffentlichungsnummer: WO 2012/150041

(56) Entgegenhaltungen:
- DE-U1-202006 009 481
- DE-U1-202006 009 481
- GB-A- 2 454 461
- US-A1- 2008 193 329
- US-A1- 2011 022 043
- US-A1- 2011 034 914
- GREGORY FRIDMAN ET AL: "Applied plasma medicine", PLASMA PROCESSES AND POLYMERS, WILEY - VCH VERLAG, DE, Bd. 5, Nr. 6, 15. August 2008 (2008-08-15) , Seiten 503-533, XP002617381, ISSN: 1612-8850, DOI: 10.1002/PPAP.200700154 [gefunden am 2008-04-16]

## Beschreibung

Die Erfindung betrifft eine Vorrichtung zur Plasmabehandlung von Oberflächen gemäß Oberbegriff des Anspruchs 1. Alle offenbarten Verfahren sind lediglich beispielhaft und fallen nicht in den Umfang der vorliegenden Erfindung.

Vorrichtungen und Verfahren zur Plasmabehandlung von Oberflächen sowie Verwendungen von Vorrichtungen zu diesem Zweck sind bekannt, z.B. aus GB2454461, DE202006009481, US2008/193329 und US2011/022043.

Typischerweise wird mithilfe einer Plasmaquelle ein Plasma erzeugt, welches auf die zu behandelnde Oberfläche einwirkt, um diese zu modifizieren, insbesondere zu sterilisieren, zu dekontaminieren und/oder zu desinfizieren. Dabei kann eine Abstandshalteeinrichtung vorgesehen sein, mithilfe derer ein vorbestimmter Abstand zwischen der Plasmaquelle und der zu behandelnden Oberfläche eingehalten wird. Hierdurch kann zum einen sichergestellt werden, dass der Abstand ausreicht, um effektiv ein Plasma zünden zu können, und dass andererseits kein zu großer Abstand gewählt wird, sodass keine wirksame Plasmabehandlung mehr möglich ist. Es hat sich herausgestellt, dass abhängig von dem konkret vorliegenden Abstand zwischen der Plasmaquelle und der zu behandelnden Oberfläche bestimmte chemische und/oder physikalische Spezies bevorzugt am Behandlungsort vorliegen. Dabei ist je nach Art der gewünschten Plasmabehandlung eine bestimmte Plasmachemie, also eine bestimmte Zusammensetzung beziehungsweise Konzentration der in dem Plasma vorliegenden Spezies, vorteilhaft. Bei bekannten Vorrichtungen, Verfahren und Verwendungen bekannter Vorrichtungen ist der Behandlungsabstand allerdings festgelegt, sodass unabhängig von der tatsächlichen Behandlung stets die gleiche Plasmachemie gegeben ist. Demnach sind bekannte Vorrichtungen, Verfahren und Verwendungen für bestimmte Behandlungsarten eher und für andere weniger geeignet. Letztlich ist es also nötig, für jede Behandlungsart insbesondere eine gesonderte Vorrichtung vorzuhalten, was teuer und umständlich ist. Umgekehrt können bestimmte Behandlungsarten nur mit eingeschränkter Wirksamkeit vorgenommen werden, wenn beispielsweise nur eine oder nur wenige Vorrichtungen zur Verfügung stehen.

Es ist daher Aufgabe der Erfindung, eine Vorrichtung zu schaffen, mithilfe derer die Plasmachemie am Behandlungsort auswählbar ist. Hierdurch können ganz verschiedene Behandlungsarten mit ein und derselben Vorrichtung durchgeführt werden. Dies vereinfacht die Plasmabehandlung, erhöht deren Wirksamkeit und/oder reduziert die Kosten.

Die Aufgabe wird gelöst, indem eine Vorrichtung mit den Merkmalen des Anspruchs 1 geschaffen wird. Diese dient der Plasmabehandlung von Oberflächen, insbesondere von Haut. Sie umfasst ein Gehäuse, eine dem Gehäuse zugeordnete Plasmaquelle und eine Abstandshalteeinrichtung, wobei die Abstandshalteeinrichtung so an dem Gehäuse und/oder der Plasmaquelle vorgesehen ist, dass zumindest bereichsweise ein Abstand zwischen der Plasmaquelle und der zu behandelnden Oberfläche eingehalten werden kann. Die Vorrichtung zeichnet sich dadurch aus, dass die Abstandshalteeinrichtung einstellbar ausgebildet ist, sodass der Abstand zwischen der Plasmaquelle und der zu behandelnden Oberfläche, nämlich der Behandlungsabstand, variiert werden kann, wobei dieser derart variiert werden kann, dass eine bevorzugte Plasmachemie am Ort der zu behandelnden Oberfläche auswählbar ist. die Variation muss also in einem bestimmten Abstandsbereich so vorgenommen werden können, dass die Konzentration wirksamer Spezies am Behandlungsort eingestellt werden kann. Dadurch ist es möglich, verschiedenste Behandlungsarten mit ein und derselben Vorrichtung effektiv durchzuführen, indem die für die jeweilige Behandlung optimale Plasmachemie ausgewählt werden kann.

Mittels der Abstandshalteeinrichtung kann ein Abstand von 4 bis 30 mm eingestellt werden. Dies sind typische Abstände, innerhalb derer sich die Konzentration der wirksamen Spezies am Behandlungsort ändert, beziehungsweise bestimmte Spezies in gewünschter Konzentration vorliegen.

Bevorzugt wird eine Vorrichtung, bei der die Abstandshalteeinrichtung ein Gewinde aufweist, das mit einem entsprechenden Gewinde der Vorrichtung kämmt. Der Abstand ist dann auf sehr einfache Weise dadurch einstellbar, dass die Abstandshalteeinrichtung relativ zu dem Rest der Vorrichtung geschwenkt beziehungsweise gedreht wird, sodass aufgrund der miteinander kämmenden Gewinde eine Änderung des Behandlungsabstands erfolgt.

Es wird auch eine Vorrichtung bevorzugt, bei der die Abstandshalteeinrichtung mindestens ein Rastelement aufweist, welches mit mindestens einem entsprechenden Rastelement der Vorrichtung zusammenwirkt, um den Abstand zu definieren. Solche miteinander korrespondierenden Rastelemente sind beispielsweise von Haarschneidemaschinen bekannt. Insbesondere ist es bei einem solchen Ausführungsbeispiel möglich, den Behandlungsabstand durch Relatiwerlagerung der Abstandseinhalteeinrichtung zum Rest der Vorrichtung - in Längsrichtung derselben gesehen - einzustellen. Bevorzugt weist die Vorrichtung, insbesondere die Abstandshalteeinrichtung eine Skala auf, anhand derer ein gewünschter Abstand beziehungsweise eine bevorzugte Plasmachemie einstellbar ist. Die Skala kann beispielsweise eine Abstandsskala sein. Es ist auch möglich, beispielsweise in Form von Piktogrammen oder durch Angabe der reaktiven Spezies die bei einem bestimmten Abstand wirksame Plasmachemie zu kennzeichnen.

Schließlich wird eine Vorrichtung bevorzugt, die eine Auswahleinrichtung aufweist, mithilfe derer eine bevorzugte Plasmachemie vorzugsweise automatisch ausgewählt werden kann. Insbesondere kann die Auswahleinrichtung bevorzugt eine gewünschte Behandlungsart wählen und die Plasmachemie beziehungsweise letztlich den Behandlungsabstand darauf abstimmen. Sie kann dabei den gewünschten Abstand beispielsweise in einem Display anzeigen, sodass dieser manuell eingestellt werden kann, oder sie kann, vorzugsweise über einen Aktuator, die Abstandshalteeinrichtung automatisch verstellen.

Weitere vorteilhafte Ausgestaltungen ergeben sich aus den Unteransprüchen.

Weiterhin wird ein Verfahren zur Plasmabehandlung von Oberflächen, insbesondere von Haut, offenbart. Das Verfahren umfasst folgende Schritte: Es wird ein Wirkmechanismus für die zu behandelnde Oberfläche ausgewählt. Der Abstand der Plasmaquelle zu der zu behandelnden Oberfläche, also der Behandlungsabstand, wird eingestellt. Die Plasmabehandlung wird durchgeführt. Das Verfahren ist somit auf viele verschiedene Behandlungsarten anwendbar, weil stets ein geeigneter Abstand und damit eine geeignete Plasmachemie am Behandlungsort ausgewählt werden kann.

Es wird ein Verfahren bevorzugt, bei dem ein Abstand zwischen ungefähr 0 und 30 mm, vorzugsweise zwischen ungefähr 4 und 20 mm eingestellt wird. Dies sind typische Abstandsbereiche, innerhalb derer bevorzugte wirksame Spezies beziehungsweise Zusammensetzungen des am Behandlungsort aktiven Plasmas ausgewählt werden können.

Besonders bevorzugt erfolgt die Auswahl des Abstandes mittels einer Auswahleinrichtung. Diese ermöglicht vorzugsweise eine automatische Abstandsauswahl, wobei ganz besonders bevorzugt folgende Schritte durchgeführt werden: Es wird mindestens ein charakteristischer Wert der zu behandelnden Oberfläche erfasst. Der erfasste Wert wird mit mindestens einem Referenzwert verglichen. Der erfasste Wert wird mindestens einem Referenzwert zugeordnet. Schließlich wird der Behandlungsabstand anhand des mindestens einen zugeordneten Referenzwerts bestimmt. Bevorzugt ist eine Datenbank mit Referenzwerten vorgesehen, sodass ein Behandlungsbedarf der zu behandelnden Oberfläche anhand eines Vergleichs des erfassten Werts mit den Referenzwerten festgestellt werden kann. Vorzugsweise sind mit diesen Angaben zu einer Plasmachemie verknüpft, die besonders wirksam in Hinblick auf die in Zusammenhang mit den Referenzwerten erforderliche Behandlung ist. Die Plasmachemie ist bevorzugt wiederum mit einem Abstandsparameter verknüpft, der einem optimalen Behandlungsabstand entspricht. Es ist bevorzugt auch möglich, dass der Abstand in Hinblick auf die gewünschte Plasmachemie berechnet wird. Alternativ kann auch direkt der Abstandsparameter mit den Referenzwerten verknüpft sein.

Es wird ein Verfahren bevorzugt, bei dem der mindestens eine erfasste Wert ein Bild der zu behandelnden Oberfläche ist, welches mit mindestens einem Referenzbild verglichen wird. Das Verfahren ist dann bevorzugt durch folgende Schritte gekennzeichnet: Es wird mindestens ein Bild der zu behandelnden Oberfläche aufgenommen. Das mindestens eine Bild wird mit mindestens einem Referenzbild verglichen. Das mindestens eine Bild wird mindestens einem Referenzbild zugeordnet. Es wird eine vorzugsweise mit dem mindestens einen Referenzbild verknüpfte Plasmachemie ausgewählt. Es wird ein Abstand bestimmt, welcher der gewählten Plasmachemie entspricht.

Vorzugsweise wird der Abstand abhängig von der gewünschten Plasmachemie anhand von mindestens einer Reaktionsgeschwindigkeit für die Bildung und/oder den Zerfall einer gewünschten Spezies sowie mindestens einer relevanten Diffusionskonstante für das Plasma berechnet. Eine solche Berechnung beruht auf der Erkenntnis, dass die in dem Plasma beziehungsweise durch das Plasma gebildeten Spezies beziehungsweise die Reaktanden zu deren Bildung bestimmte Diffusionskonstanten aufweisen. Auch ist die am Behandlungsort gewünschte Plasmachemie gekennzeichnet durch Reaktionsgeschwindigkeiten, mit denen die wirksamen Spezies gebildet werden beziehungsweise wieder zerfallen. Aus den Reaktionsgeschwindigkeiten einerseits und den Diffusionskonstanten andererseits ergibt sich ein Abstand von der Plasmaquelle, an dem eine gewünschte Plasmachemie, also eine gewünschte Konzentration bestimmter reaktiver Spezies vorliegt. Entsprechend kann der Abstand berechnet werden, wenn die Diffusionskonstanten und Reaktionsgeschwindigkeiten bekannt sind.

Weitere vorteilhafte Ausgestaltungen ergeben sich aus den Unteransprüchen.

Die Vorrichtung wird bevorzugt verwendet zur Behandlung von Wunden, Hautirritationen, Infektionen, Insektenstichen, Pilzbefall der Füße, insbesondere Fußpilz, Akne, Herpes, Pickel, Verbrennungen, Ohrinfektionen, Ausschlägen, insbesondere Windelausschlag, Hitzebläschen, Läusebefall, insbesondere von Kopf oder Körper, Flohbefall, anderer Befall durch Wirbellose, Schuppen, empfindlicher Haut, Pilzbefall der Nägel, Psoriasis, Fieberbläschen. Sie wird auch oder alternativ verwendet zur Verhinderung, Entfernung, Modifizierung oder Reduzierung von Körpergeruch, beispielsweise Fußgeruch, Achselgeruch, durch Inkontinenz verursachter Geruch, Geruch des Intimbereichs und/oder aus dem Körperinneren entweichender Geruch.

Die Erfindung wird im Folgenden anhand der Zeichnung näher erläutert. Es zeigen:
- Figur 1: eine schematische Ansicht eines ersten Ausführungsbeispiels einer Vorrichtung zur Plasmabehandlung von Oberflächen;
- Figur 2: ein zweites Ausführungsbeispiel einer Vorrichtung;
- Figur 3: ein drittes Ausführungsbeispiel einer Vorrichtung, und
- Figur 4: ein viertes Ausführungsbeispiel einer Vorrichtung.

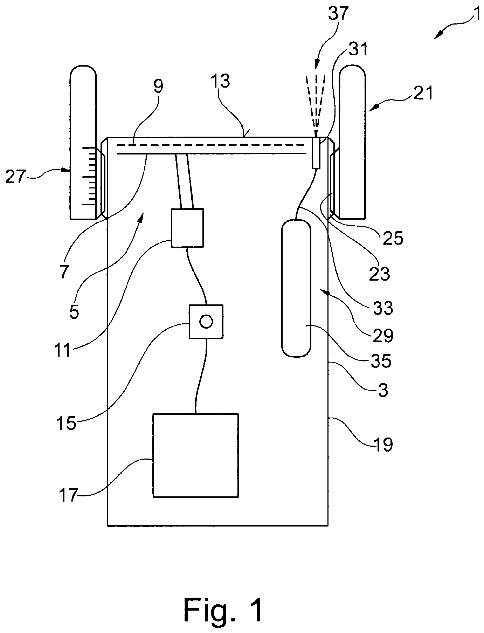

Figur 1 zeigt eine schematische Darstellung einer Vorrichtung 1 zur Plasmabehandlung von Oberflächen, insbesondere von Haut. Sie umfasst einen Grundkörper, hier ein Gehäuse 3. Vorzugsweise in dieses integriert oder an diesem vorgesehen ist eine Plasmaquelle 5, durch die ein Plasma zur Oberflächenbehandlung erzeugbar ist.

Die Plasmaquelle 5 kann verschieden aufgebaut sein: Die Plasmaquelle umfasst zwei Elektroden, von denen eine mit einer Wechselspannung beaufschlagt wird, wobei die andere vorzugsweise geerdet ist. Um die Sicherheit eines Benutzers zu gewährleisten, ist dabei vorzugsweise die geerdete Elektrode an einer der zu behandelnden Fläche zugewandten Oberfläche vorgesehen. Die beiden Elektroden sind durch ein Dielektrikum voneinander beabstandet. Zwischen den Elektroden wird eine Entladung gezündet, sodass auf der Seite der Elektrode, welche der zu behandelnden Oberfläche zugewandt ist, ein Plasma gebildet wird, das auf die zu behandelnde Oberfläche einwirkt. Dies entspricht dem Prinzip der Oberflächenmikroentladung (Surface Micro Discharge - SMD).

Es ist bevorzugt auch möglich, eine Plasmaquelle vorzusehen, bei der zumindest die der zu behandelnden Oberfläche zugewandte Elektrode in ein Dielektrikum eingebettet ist. Dabei ist sie so nah an der Oberfläche des Dielektrikums vorgesehen, dass ein Plasma auf dieser Oberfläche gebildet wird, wenn eine Entladung zwischen den Elektroden gezündet wird. Vorzugsweise ist auch in diesem Fall die Elektrode geerdet, welche der zu behandelnden Oberfläche zugewandt ist. An die nicht geerdete Elektrode wird bevorzugt eine Wechselspannung, besonders bevorzugt eine hochfrequente Wechselspannung angelegt. Bevorzugt ist auch die nicht der zu behandelnden Oberfläche zugewandte Elektrode in ein Dielektrikum eingebettet. In diesem Fall ist keine der Elektroden frei zugänglich. Insbesondere die Einbettung der Elektrode, welche der zu behandelnden Oberfläche zugewandt ist, entspricht im Prinzip der selbststerilisierenden Oberfläche (self sterilising surface - SSS).

Bei den in den Figuren dargestellten Ausführungsbeispielen ist jeweils eine Plasmaquelle schematisch angedeutet, die dem Prinzip der selbststerilisierenden Oberfläche genügt. Dies entspricht einer bevorzugten Ausführungsform; gleichwohl ist es ohne Weiteres auch möglich, eine andere Plasmaquelle bevorzugt vorzusehen. Insbesondere wenn die Vorrichtung 1 der Behandlung von Haut dient, ist bevorzugt eine Plasmaquelle 5 vorgesehen, die nach dem Prinzip der Oberflächenmikroentladung oder besonders bevorzugt der selbststerilisierenden Oberfläche funktioniert. In diesem Fall wird nämlich eine Beaufschlagung der Haut mit elektrischem Strom vermieden, wodurch die Plasmabehandlung jedenfalls angenehmer, gegebenenfalls auch ungefährlicher wird.

Bei dem dargestellten Ausführungsbeispiel umfasst die Plasmaquelle 5 eine erste Elektrode 7 und eine zweite Elektrode 9. Beide Elektroden 7, 9 sind elektrisch mit einer Spannungsquelle 11 verbunden, sodass bevorzugt die erste Elektrode 7 mit einer Hochspannung geeigneter Frequenz beaufschlagt wird, wobei die zweite Elektrode 9 bevorzugt geerdet ist. Beide Elektroden 7, 9 sind bevorzugt in ein Dielektrikum eingebettet, wobei die zweite Elektrode 9 etwas unterhalb einer Oberfläche 13 angeordnet ist, in deren Bereich ein Plasma erzeugt wird, wenn eine Entladung zwischen den Elektroden 7, 9 gezündet wird.

Bevorzugt umfasst die Vorrichtung 1 einen Schalter 15, über den die Plasmaquelle 5 aktivierbar beziehungsweise deaktivierbar ist. Sie umfasst weiterhin bevorzugt eine Energiequelle 17, durch welche die Spannungsquelle 11 mit Energie versorgbar ist. Die Energiequelle 17 kann - wie hier beispielhaft dargestellt - als Batterie, Akkumulator oder sonstige dem Gehäuse 3 zugeordnete Energiequelle ausgebildet sein. Bevorzugt ist es aber auch möglich, einen Anschluss für eine externe Energiequelle, beispielsweise einen Stecker, vorzusehen. Ebenfalls ist es möglich, die Plasmaquelle 5 über eine Energieerntevorrichtung (energy-harvesting device) mit Energie zu versorgen. Hier kommen beispielsweise Piezokristalle, in Spulen verschiebliche Permanentmagnete, allgemein induktive Vorrichtungen, Vorrichtungen, die auf dem Prinzip der Thermoelektrizität, beispielsweise dem Seebeck-Effekt, Peltier-Effekt und/oder Thomson-Effekt, basieren, Solarpaneele und zahlreiche andere an sich bekannte Vorrichtungen in Betracht. Diese sind teilweise bevorzugt in die Spannungsquelle 11 integriert oder ersetzen diese. Beispielsweise kann ein Piezokristall vorgesehen sein, welcher zugleich als Spannungsquelle und als Energiequelle dient.

Insbesondere wird die Energie zur Erzeugung des Plasmas bevorzugt einer Bewegung der Vorrichtung 1 entnommen, wobei diese eine Schwerpunktbewegung der Vorrichtung 1, aber auch eine Bewegung von Teilen der Vorrichtung 1 relativ zueinander sein kann.

Die Vorrichtung 1 ist bevorzugt als mobile, insbesondere tragbare Vorrichtung, ganz besonders als Handgerät ausgebildet. In diesem Fall weist sie vorzugsweise einen Griffbereich 19 auf, in dem besonders bevorzugt eine rutschfeste Oberfläche vorgesehen ist, sodass ein Benutzer die Vorrichtung 1 sicher greifen kann.

Bei einem anderen Ausführungsbeispiel kann die Vorrichtung 1 bevorzugt als fest installierte oder jedenfalls nicht zum einfachen Transport bestimmte Vorrichtung ausgebildet sein. Denkbar ist beispielsweise eine in einer Behandlungspraxis vorgesehene Vorrichtung.

Die Vorrichtung 1 ist insbesondere verwendbar zur Behandlung von Wunden, Hautirritationen, Infektionen, Insektenstichen, Pilzbefall der Füße, insbesondere Fußpilz, Akne, Herpes, Pickel, Verbrennungen, Infektionen, insbesondere Ohrinfektionen, Ausschlägen, insbesondere Windelausschlag, Hitzebläschen, Fiederbläschen, Läusebefall, insbesondere Läusebefall von Kopf oder Körper, Flohbefall, anderer Befall durch Wirbellose, Schuppen, empfindlicher Haut, Pilzbefall der Nägel und/oder Psoriasis. Sie kann bevorzugt auch verwendet werden zur Verhinderung, Entfernung, Überdeckung beziehungsweise Maskierung, Modifizierung oder Reduzierung von Körpergeruch. Umfasst sind beispielsweise Fußgeruch, Achselgeruch, durch Inkontinenz verursachter Geruch, Geruch des Intimbereichs und/oder aus dem Körperinneren entweichender Geruch.

In einem bevorzugten Ausführungsbeispiel ist die Vorrichtung zur Verwendung in einer warmen, feuchten Umgebung, beispielsweise in einem Schwimmbad oder einer Sauna vorgesehen. Hier wird sie vorzugsweise zur Vermeidung oder Behandlung von Fußpilz oder anderem Pilzbefall der Haut eingesetzt.

Die Vorrichtung ist vorzugsweise auch zur Reduzierung oder Verhinderung von Zahnschmerzen einsetzbar.

Dabei zeigt sich, dass für die verschiedenen Verwendungen der Vorrichtung verschiedene chemische Spezies wirksam beziehungsweise geeignet sind, die von dem Plasma umfasst sind oder durch dieses gebildet werden.

Dabei schließt der Begriff Plasma nicht nur das Plasma in engerem Sinne, also den so genannten "vierten Aggregatzustand" ein, der verschiedennamig geladene Teilchen in einem Volumen umfasst, welches insgesamt quasi-neutral ist, sondern der Begriff umfasst auch durch das Plasma im engeren Sinne oder mit diesem gemeinsam gebildete ungeladene, reaktive Spezies, beispielsweise angeregte Atome oder Moleküle, freie Radikale oder weitere aktive Spezies.

Die Vorrichtung 1 ist zur Anwendung für die Zellregeneration geeignet. In diesem Fall und auch gegen Hautirritationen sind beispielsweise Stickoxide, NO beziehungsweise NOₓ besonders wirksam.

Für eine bakterizide Wirkung ist das Hydroxyl-Radikal beziehungsweise OH-Radikal besonders wirksam. Hierbei ist zu beachten, dass dieses in hohen Konzentrationen toxisch ist. Gegebenenfalls muss also bezüglich der gewünschten Konzentration ein Kompromiss zwischen effektiver Behandlung und Toxizität eingegangen werden, um die für die Behandlung geeignete Konzentration zu bestimmen.

Ebenfalls für die bakterizide Wirkung des Plasmas, allerdings auch zur Bekämpfung unerwünschter Gerüche ist Ozon, O₃, besonders wirksam.

In dieser Weise können den verschiedenen Behandlungsarten verschiedene reaktive Spezies zugeordnet werden, die vorzugsweise am Behandlungsort in erhöhter Konzentration vorliegen können.

Abhängig von den Reaktionsgeschwindigkeiten, mit denen dieses Spezies entstehen und wieder zerfallen, und weiter abhängig von den konkret vorliegenden Transportmechanismen, mit denen das Plasma von der Oberfläche 13, an der es erzeugt wird, auf die zu behandelnde Oberfläche transportiert wird, ergeben sich verschiedene Abstände von der Oberfläche 13, bei denen verschiedene Spezies in erhöhter Konzentration vorliegen. Als Transportmechanismus kommt insbesondere Diffusion in Betracht. Insoweit sind hier die Diffusionskonstanten der Reaktanden beziehungsweise der sich bildenden oder zerfallenden Spezies relevant. Gegebenenfalls kommt auch Konvektion in Betracht, oder es wird bei bestimmten Ausführungsbeispielen eine Strömung des Plasmas erzeugt, die dann entsprechend bei der Wahl des Behandlungsabstands zu berücksichtigen ist.

Jedenfalls kann quasi eine ortsabhängige Plasmachemie angegeben werden, sodass einer effektiv durchzuführenden Behandlung ein bestimmter Behandlungsabstand, nämlich ein bestimmter Abstand der Oberfläche 13 von der zu behandelnden Oberfläche entspricht. Beispielsweise liegen bei einem bestimmten Abstand reaktive Stickstoff-Spezies in besonders günstiger Konzentration vor.

Die hier relevanten Abstände liegen typischerweise auf einer Längenskala von 4 mm bis 20 mm.

Um den gewünschten Abstand einstellen zu können, umfasst die Vorrichtung 1 eine Abstandshalteeinrichtung 21, mittels derer ein Abstand zwischen der Plasmaquelle 5 und der zu behandelnden Oberfläche einhaltbar ist. Diese ist einstellbar ausgebildet, sodass der Abstand variiert werden kann. Ein Abstand von 4 bis 30 mm ist einstellbar. Dadurch ist sichergestellt, dass eine geeignete Plasmachemie für die gewünschte Behandlungsart ausgewählt werden

Um eine Variation des Behandlungsabstandes zu bewirken, umfasst die Abstandshalteeinrichtung 21 bei dem dargestellten Ausführungsbeispiel ein Innengewinde 23, welches mit einem Außengewinde 25 kämmt, das hier an dem Gehäuse 3 vorgesehen ist. Die Abstandshalteeinrichtung 21 und das Gehäuse 3 können in diesem Fall relativ zueinander gedreht werden, um den Behandlungsabstand einzustellen.

Bei einem anderen Ausführungsbeispiel umfasst die Abstandshalteeinrichtung 21 mindestens ein Rastelement, das mit mindestens einem entsprechenden Rastelement der Vorrichtung, insbesondere einem am Gehäuse 3 vorgesehenen Rastelement zusammenwirkt, um den Abstand zu definieren.

Bei wieder einem anderen Ausführungsbeispiel ist es möglich, dass die Abstandshalteeinrichtung 21 relativ zu dem Gehäuse 3 - in Längsrichtung der Vorrichtung 1 gesehen - verlagert werden kann, wobei die Reibungsverhältnisse zwischen der Abstandshalteeinrichtung 21 und dem Gehäuse 3 so gewählt sind, dass feinfühlig und sicher ein bestimmter Abstand einstellbar ist, ohne dass es definierter Rastpositionen bedarf. Der Abstand ist dann kontinuierlich variabel.

Besonders bevorzugt weist die Vorrichtung 1 eine Skala auf, anhand derer ein gewünschter Abstand beziehungsweise eine bevorzugte Plasmachemie einstellbar ist. Bei dem dargestellten Ausführungsbeispiel ist eine Skala 27 an der Abstandshalteeinrichtung 21 vorgesehen. Die Skala 27 kann den Behandlungsabstand wiedergeben, wobei in diesem Fall vorzugsweise eine Tabelle vorgesehen ist, welche eine gewünschte Behandlungsart beziehungsweise die dazu passende Plasmachemie einem bestimmten Abstand zuordnet. Es ist aber auch möglich, statt einer numerischen Skala 27 Piktogramme vorzusehen, aus denen gewünschte Behandlungsarten oder eine bestimmte Plasmachemie hervorgehen.

Das mithilfe der Plasmaquelle 5 erzeugte Plasma hat die Eigenschaft, in das Gewebe textiler Materialien einzudringen beziehungsweise textile Materialien durchdringen zu können. Daher ist eine Anwendung der Vorrichtung 1 auch durch Kleidung hindurch möglich. Gegebenenfalls muss dann allerdings der durch die Kleidung bewirkte Abstand zu dem Abstand hinzugerechnet werden, der durch die Abstandshalteeinrichtung 21 vorgegeben ist.

Bei einer Behandlung textiler Oberflächen zeigt sich, dass das Plasma aufgrund seiner Fähigkeit, in deren Gewebe einzudringen, eine gewisse Tiefenwirkung entfaltet. Der Begriff "Oberfläche" schließt also in diesem Fall ein gewisses, mitbehandeltes Volumen in dem textilen Material ein. Auch bei der Behandlung von Haut zeigt sich, dass eine gewisse Tiefenwirkung dadurch existiert, dass parakrine Effekte beziehungsweise eine interzelluläre Kommunikation in den Wirkmechanismus einbezogen werden. Ein bestimmter Behandlungseffekt wird dabei quasi von Zelle zu Zelle weitergegeben, sodass er auch jenseits der eigentlich behandelten Oberfläche wirksam ist.

Bevorzugt umfasst die Vorrichtung 1 eine Applikationseinrichtung 29, durch die mindestens ein Zusatzstoff auf die zu behandelnde Oberfläche aufbringbar ist. Bei dem dargestellten Ausführungsbeispiel ist die Applikationseinrichtung 29 als Spray ausgebildet, wobei sie eine Düse 31, eine Zuleitung 33 und einen Vorratsbehälter 35 umfasst. Die Düse 31 ist über die Zuleitung 33 mit dem Vorratsbehälter 35 verbunden. Dieser ist vorzugsweise nachfüllbar und/oder austauschbar an beziehungsweise in dem Gehäuse 3 vorgesehen. Nicht dargestellt ist eine Pumpvorrichtung, über die vorzugsweise der mindestens eine Zusatzstoff aus dem Vorratsbehälter 35 über die Zuleitung 33 zu der Düse 31 transportiert und aus dieser ausgetragen werden kann. Die Pumpvorrichtung kann automatisch, insbesondere elektrisch, oder auch manuell, insbesondere nach Art eines Pumpsprays, betrieben werden. Dabei ist es möglich, die Pumpvorrichtung vor, nach oder gleichzeitig mit der Plasmaquelle 5 zu aktivieren. Schematisch dargestellt ist ein Sprühnebel 37, der aus der Düse 31 ausgetragen wird.

Bei einem anderen Ausführungsbeispiel kann die Applikationseinrichtung als Rolleinrichtung, beispielsweise nach Art eines Deorollers, oder als Stick- beziehungsweise Stiftvorrichtung, insbesondere nach Art eines Deostifts oder -sticks ausgebildet sein.

Als Zusatzstoff kommt bevorzugt ein Stoff infrage, der einen Geruch auf der zu behandelnden Oberfläche entfernt, modifiziert, maskiert, reduziert, oder dessen Bildung verhindert. Insoweit kann der Zusatzstoff beispielsweise ein Parfum oder Deodorant umfassen. Er kann auch ein Antitranspirant umfassen, welches eine Schweißbildung verhindert. Zugleich ist es möglich, dass der Zusatzstoff einen "technischen" Geruch des Plasmas überdeckt.

Bei einem anderen bevorzugten Ausführungsbeispiel ist der Zusatzstoff so ausgewählt, dass er die bakterizide Wirkung des Plasmas unterstützt, das Bakterienwachstum hemmt, desinfizierend wirkt, antiviral wirkt und/oder dekontaminierende Eigenschaften in Hinblick auf die zu behandelnde Oberfläche aufweist.

Schließlich umfasst der Zusatzstoff bevorzugt einen Stoff, der zur Wundbehandlung geeignet ist, Hautirritationen lindert, zur Behandlung von Insektenstichen geeignet ist, der Pilzbehandlung dient, insbesondere fungizid ist, zur Behandlung von Akne, Herpes oder Pickeln geeignet ist, zur Behandlung von Verbrennungen geeignet ist, Ohrinfektionen lindert oder bekämpft, zur Linderung oder Bekämpfung von Ausschlägen, insbesondere Windelausschlag, geeignet ist, zur Behandlung von Hitzebläschen, Fieberbläschen oder sonstiger Blasenbildung der Haut geeignet ist, zur Bekämpfung von Läusen und/oder Flöhen beziehungsweise anderen Wirbellosen geeignet ist, eine Antischuppen-Eigenschaft aufweist, zur Behandlung empfindlicher Haut geeignet ist, Schuppenflechte vorbeugt, lindert oder bekämpft, Haarwuchs fördert oder inhibiert, haarentfernend wirkt, und/oder andere Eigenschaften hat, die in Zusammenhang mit der zu behandelnden Oberfläche, insbesondere in Zusammenhang mit der Behandlung von Haut, wünschenswert ist. Dabei unterstützt der Zusatzstoff bevorzugt den mit der gewählten Behandlungsart erzielten Effekt.

Selbstverständlich ist es möglich, verschiedene Zusatzstoffe in Kombination miteinander, nacheinander, alternativ zueinander und/oder in beliebiger Mischung zu verwenden.

Durch die Plasmaquelle 5 wird bevorzugt ein kaltes Plasma gebildet, sodass die zu behandelnde Oberfläche, insbesondere Haut, keiner hohen thermischen Belastung ausgesetzt ist. Besonders bevorzugt wird bei der Plasmaerzeugung auch wenig UV-Strahlung erzeugt, sodass die zu behandelnde Oberfläche hierdurch nicht übermäßig belastet wird. Bei manchen Behandlungsarten kann jedoch eine Bestrahlung mit UV-Strahlung wünschenswert sein. In diesem Fall ist vorgesehen, dass die UV-Intensität der Plasmaquelle 5 vorzugsweise erhöht, insbesondere an eine gewünschte Intensität angepasst werden kann.

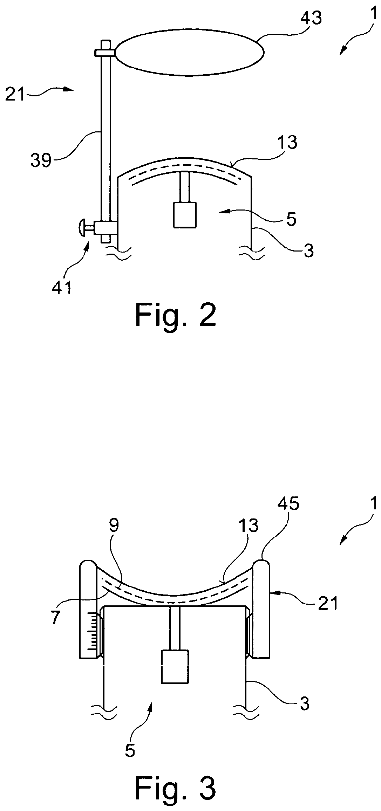

Figur 2 zeigt eine schematische Darstellung eines zweiten Ausführungsbeispiels der Vorrichtung. Gleiche und funktionsgleiche Elemente sind mit gleichen Bezugszeichen versehen, sodass insofern auf die vorangegangene Beschreibung verwiesen wird.

Die Oberfläche 13 ist hier gekrümmt, insbesondere konvex, ausgebildet. Eine gekrümmte Oberfläche 13 hat den Vorteil, dass sie insbesondere der Krümmung einer zu behandelnden Oberfläche angepasst werden kann. Auch bei unregelmäßig gekrümmten zu behandelnden Oberflächen existiert so stets mindestens ein Punkt, an dem ein gewünschter Behandlungsabstand vorliegt.

Abhängig von der Krümmung der zu behandelnden Oberfläche einerseits und der Krümmung der Oberfläche 13 andererseits existiert eine ganze Schar von Behandlungsabständen, sodass verschiedene Wirkmechanismen zugleich realisierbar sind. Wird beispielsweise bei dem in Figur 2 dargestellten Ausführungsbeispiel der höchste Punkt der Oberfläche 13 der zu behandelnden Oberfläche so weit angenähert, dass in diesem Bereich eine Dissoziation von Molekülen durch heiße Elektronen stattfindet, existiert radial weiter außerhalb ein Bereich, in dem die bakterizide Wirkung des Plasmas überwiegt. In wieder einem anderen radialen Abstand zum höchsten Punkt der Oberfläche 13 überwiegt die regenerative Wirkung des Plasmas auf Hautzellen, beziehungsweise die Wirkung gegen Hautirritationen. Es zeigt sich damit, dass eine gekrümmte Oberfläche - sei sie konvex, konkav oder unregelmäßig gekrümmt - eine Vielzahl möglicher Wirkmechanismen zugleich umfassen kann. Wird die zu behandelnde Oberfläche dann mit der Vorrichtung 1 überstrichen, kann so an jedem Punkt derselben jeder der vorliegenden Behandlungsmechanismen wirksam werden.

Bei dem in Figur 2 dargestellten Ausführungsbeispiel ist eine sehr einfache Abstandshalteeinrichtung 21 vorgesehen. Diese umfasst hier lediglich einen Stab 39, der an dem Gehäuse 3 vorzugsweise mithilfe einer Klemmvorrichtung 41 verschieblich angeordnet ist. Letztlich genügt der Stab 39, um einen definierten Abstand jedenfalls zum höchsten Punkt der Oberfläche 13 zu gewährleisten. Bevorzugt ist aber an dem Stab 39 ein Abstandsring 43 vorgesehen, der bevorzugt an der zu behandelnden Oberfläche angelegt wird, um den Behandlungsabstand zu definieren.

Figur 3 zeigt eine schematische Darstellung eines dritten Ausführungsbeispiels der Vorrichtung 1. Gleiche und funktionsgleiche Elemente sind mit den gleichen Bezugszeichen versehen, sodass insofern auf die vorangegangene Beschreibung verwiesen wird. Die Oberfläche 13 ist hier flexibel und/oder elastisch ausgebildet und vorzugsweise so mit der Abstandshalteeinrichtung 21 verbunden, dass sie mit dieser zusammen verlagert beziehungsweise durch diese verformt werden kann. Dazu ist die Oberfläche 13 besonders bevorzugt mit ihrem tiefsten Punkt an dem Gehäuse 3 festgelegt. Wird die Abstandshalteeinrichtung 21 verlagert, verformt sich die Oberfläche 13 und ändert dabei insbesondere ihre Krümmung. Vorzugsweise sind die Elektroden 7,9 in die Oberfläche 13 integriert.

Die Oberfläche 13 ist hier konkav ausgebildet. Wird eine zu behandelnde Oberfläche auf einen oberen Rand 45 der Abstandshalteeinrichtung 21 aufgelegt, entsteht so ein geschlossenes Volumen, in dem das Plasma gebildet wird. Dies ist besonders vorteilhaft, weil keinerlei Störungen von außen auf das Plasma einwirken, beispielsweise keine Luftströmungen. Es ist so eine besonders intensive Plasmabehandlung möglich. Gegebenenfalls kann daher auch die Leistung der Plasmaquelle 5 reduziert werden, was insbesondere dann vorteilhaft ist, wenn empfindliche Haut behandelt wird. Wie schon in Zusammenhang mit Figur 2 angesprochen, werden auch bei einer konkaven Oberfläche 13 abhängig von der Krümmung der zu behandelnden Oberfläche verschiedene Wirkmechanismen in verschiedenen Bereichen der Oberfläche 13 realisiert.

Abweichend von der Darstellung gemäß Figur 3 ist es alternativ oder zusätzlich möglich, den tiefsten Punkt der flexiblen beziehungsweise elastischen Oberfläche 13 relativ zum Gehäuse 3 anzuheben und/oder abzusenken. Hierzu kann beispielsweise eine Art Stempel oder ein anderer geeigneter Aktuator vorgesehen sein. Da auf diese Weise der tiefste Punkt der Oberfläche 13 bezüglich seines Abstands zu einer zu behandelnden Oberfläche variiert werden kann, ist es nicht zwingend erforderlich, dass dann die Abstandshalteeinrichtung 21 variabel ausgebildet ist. Im Gegenteil realisiert der einstellbare tiefste Punkt in Zusammenwirkung mit einer gegebenenfalls fixierten Abstandshalteeinrichtung 21 wiederum eine Abstandshalteeinrichtung, indem ein Behandlungsabstand zwischen dem tiefsten Punkt und der zu behandelnden Oberfläche variabel definierbar ist.

Vorzugsweise umfasst die Oberfläche 13 ein gummiartiges elastisches Material, besonders bevorzugt Gummi. Die vorzugsweise in das flexible und/oder elastische Material integrierten Elektroden 7,9 sind besonders bevorzugt auch selbst flexibel und/oder elastisch ausgebildet.

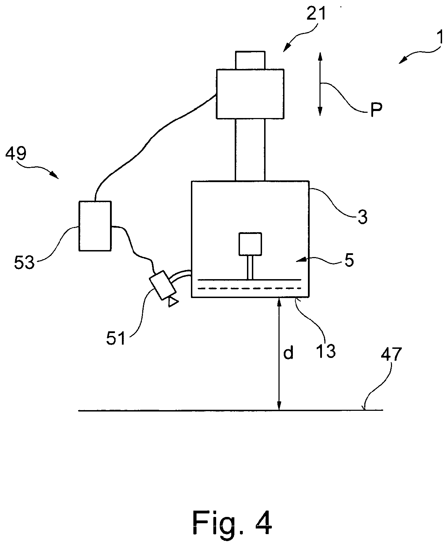

Figur 4 zeigt eine schematische Ansicht eines vierten Ausführungsbeispiels der Vorrichtung 1. Gleiche und funktionsgleiche Elemente sind mit den gleichen Bezugszeichen versehen, sodass insofern auf die vorangegangene Beschreibung verwiesen wird. Die Vorrichtung 1 ist hier beispielhaft als eine nicht-transportable, vorzugsweise fest installierte Vorrichtung ausgebildet. Alle in Zusammenhang mit der hier dargestellten Vorrichtung 1 im Folgenden beschriebenen Merkmale und Funktionen sind jedoch bei anderen bevorzugten Ausführungsbeispielen auch in mobilen, tragbaren Vorrichtungen, insbesondere in Handgeräten, vorgesehen.

Die Vorrichtung 1 dient der Behandlung einer Oberfläche 47, wobei hier der Abstand zwischen der Oberfläche 13 und der Oberfläche 47, also der Behandlungsabstand, mit d gekennzeichnet ist.

Es ist eine Abstandshalteeinrichtung 21 vorgesehen, mittels derer das Gehäuse 3, welcher die Plasmaquelle 5 umfasst, hier in Richtung des Pfeils P auf die Oberfläche 47 zu beziehungsweise von dieser weg bewegt werden kann, um einen gewünschten Behandlungsabstand d einzustellen.

Wesentlich an dem dargestellten Ausführungsbeispiel der Vorrichtung 1 ist, dass diese eine Auswahleinrichtung 49 umfasst, mittels derer der Behandlungsabstand d auswählbar ist.

Dazu umfasst die Auswahleinrichtung 49 bevorzugt einen Sensor, hier eine Kamera 51, zur Erfassung von Eigenschaften der zu behandelnden Oberfläche 47. Der Sensor ist bei anderen bevorzugten Ausführungsbeispielen als Messfühler, beispielsweise als Widerstandsmessgerät, oder auch als "Schnüffler" ausgebildet, beispielsweise als Gaschromatograph oder Ionenmobilitätsmesszelle, wobei er bevorzugt auf der Oberfläche 47 angeordnete Moleküle oder sonstige Partikel absaugen und deren Identität bestimmen kann. Jedenfalls ist mithilfe des Sensors ein charakteristischer Wert der Oberfläche 47 erfassbar.

Der Sensor, hier die Kamera 51, ist vorzugsweise mit einer Vergleichseinrichtung 53, beispielsweise einem Rechner, verbunden. Dieser wertet die von dem Sensor kommenden Daten in Hinblick auf Eigenschaften der Oberfläche 47 und eine entsprechend dieser Eigenschaften zu wählenden Behandlungsart aus. Diese ist vorzugsweise mit einer bestimmten Plasmachemie verknüpft, die durch die Vergleichseinrichtung 53 bestimmt wird. Vorzugsweise berechnet die Vergleichseinrichtung 53 einen Abstand d, der in Hinblick auf die ausgewählte Plasmachemie geeignet ist. Es ist auch möglich, dass mit einer durch die Vergleichseinrichtung 53 gewählten Behandlungsart zugleich ein bestimmter Abstand d verknüpft ist, ohne dass eine Berechnung des Abstands auf der Grundlage der Plasmachemie nötig ist.

Die Auswertung der Daten erfolgt in der Vergleichseinrichtung 23 bevorzugt durch Vergleich des mindestens einen aufgenommenen Werts mit mindestens einem Referenzwert, vorzugsweise einer Vielzahl von Referenzwerten. Diese sind vorzugsweise charakteristisch für bestimmte Zustände der zu behandelnden Oberfläche, welche einer bestimmten Behandlungsart bedürfen. Die Vergleichseinrichtung 53 ordnet dem erfassten Wert mindestens einen Referenzwert zu, wodurch ein bestimmter Zustand der zu behandelnden Oberfläche identifiziert beziehungsweise eine Behandlungsart festgelegt wird. Mit dieser kann eine gewünschte Plasmachemie verknüpft sein, mithilfe derer der Abstand d berechnet werden kann. Bei einem anderen Ausführungsbeispiel ist jedem Referenzwert unmittelbar ein Abstand d zugeordnet, sodass dieser nicht erst berechnet werden muss.

Die Vergleichseinrichtung 53 ist vorzugsweise mit der Abstandshalteeinrichtung 21 verbunden, sodass der gewählte oder berechnete Abstand d automatisch einstellbar ist.

Bei einem anderen Ausführungsbeispiel ist es möglich, dass die Vergleichseinrichtung 53 den gewählten oder berechneten Abstand d anzeigt, sodass er mithilfe der Abstandshalteeinrichtung 21 manuell eingestellt werden kann.

Wird die Behandlungsart - wie bei dem in Figur 4 dargestellten Ausführungsbeispiel - optisch mithilfe einer Kamera 51 bestimmt, werden bevorzugt folgende Verfahrensschritte durchgeführt:
Die Kamera 51 nimmt mindestens ein Bild der Oberfläche 47 auf. Dieses wird in der Vergleichseinrichtung 53 mit mindestens einem Referenzbild, vorzugsweise mit einer Schar in einer Datenbank gespeicherten Referenzbilder, verglichen. Als Ergebnis dieses Vergleichs wird dem durch die Kamera 51 aufgenommenen Bild mindestens ein passendes Referenzbild zugeordnet. Beispielsweise zeigt ein solches Referenzbild einen Insektenstich, eine Hautirritation oder andere behandlungsbedürftige Eigenschaften der Oberfläche 47.

Mit den in der Datenbank hinterlegten Referenzbilden sind vorzugsweise Behandlungsarten, Abstände d und/oder Parameter einer Plasmachemie verknüpft, die für eine Behandlung der auf dem jeweiligen Referenzbild dargestellten Verfassung der Oberfläche 47 optimal sind.

Ist mit dem Referenzbild unmittelbar ein Abstand d verknüpft, kann dieser direkt ausgewählt und vorzugsweise an die Abstandshalteeinrichtung 21 weitergegeben werden. Alternativ kann ein entsprechendes Signal an die Abstandshalteeinrichtung 21 zur Einstellung des gewählten Abstands weitergegeben werden.

Ist eine Behandlungsart mit dem Referenzbild verknüpft, ist diese wiederum entweder mit einer bestimmten Plasmachemie oder unmittelbar mit einem bestimmten bevorzugten Abstand d verknüpft.

Ist schließlich das Referenzbild mit einer bestimmten Plasmachemie verknüpft, ist diese wiederum entweder mit einem bestimmten Abstand verknüpft, oder der Abstand wird bevorzugt abhängig von der gewünschten Plasmachemie anhand von mindestens einer Reaktionsgeschwindigkeit für die Bildung und/oder den Zerfall einer gewünschten Spezies sowie mindestens einer relevanten Diffusionskonstante für das Plasma berechnet.

Es ist selbstverständlich, dass die hier in Hinblick auf die Bestimmung des Abstands d aus dem Vergleich der aufgenommenen Bilder mit Referenzbildern ausgeführten Möglichkeiten beziehungsweise Ausführungsformen des Verfahrens genauso anwendbar sind, wenn statt der Bilder andere charakteristische Werte mit entsprechenden Referenzwerten verglichen werden.

Besonders bevorzugt ist es mithilfe der Auswahleinrichtung 49 auch möglich, die Behandlungsintensität, also beispielsweise die Konzentration des auf die Oberfläche 47 einwirkenden Plasmas und/oder die Behandlungsdauer, anhand der über die Oberfläche 47 gewonnenen Daten auszuwählen.

Es ist auch möglich, in Zusammenhang mit der Auswahleinrichtung 49 mehrere Sensoren, beispielsweise eine Kamera 51 und einen Messfühler, miteinander zu kombinieren. Hierdurch kann noch genauer eine gewünschte Behandlungsart ausgewählt werden.

Besonders bevorzugt werden mit den Referenzwerten Daten einer zur Behandlung gewünschten Plasmachemie verknüpft, weil diese unabhängig von der konkreten Ausgestaltung der Vorrichtung 1 ist. Eine entsprechende Datenbank kann dann von einer Vielzahl von Vorrichtungen 1 genutzt werden.

Demgegenüber ist ein konkreter Abstand d spezifisch für die konkrete Vorrichtung 1, weil in diesen gegebenenfalls bestimmte Transportmechanismen von der Oberfläche 13 zur Oberfläche 47, die Intensität der Plasmaquelle 5 beziehungsweise andere Parameter der Vorrichtung 1 eingehen.

Eine optische Diagnose der Oberfläche 47 wird bevorzugt, weil diese berührungslos und sehr schnell erfolgt.

Wesentlich für das Verfahren zur Plasmabehandlung von Oberflächen, insbesondere von Haut, ist, dass ein Wirkmechanismus für die zu behandelnde Oberfläche ausgewählt wird, dass ein Abstand der Plasmaquelle zu der zu behandelnden Oberfläche eingestellt wird, und dass die Plasmabehandlung durchgeführt wird.

Schließlich sind Merkmale des Verfahrens auch aufgrund der Beschreibung der Eigenschaften der hier vorgestellten Ausführungsbeispiele der Vorrichtung 1 deutlich geworden.

Insgesamt zeigt sich, dass es mithilfe der Vorrichtung verschiedenste Plasmabehandlungen von Oberflächen mithilfe desselben Geräts, insbesondere mithilfe eines mobilen, in der Hand tragbaren Geräts, durchzuführen, wobei jeweils eine optimale Plasmachemie für die gewünschte Behandlungsart eingestellt werden kann.

## Patentansprüche

1. Vorrichtung zur Plasmabehandlung von Oberflächen (47), insbesondere von Haut, mit
- einem Gehäuse (3);
- einer dem Gehäuse (3) zugeordneten Plasmaquelle (5), und
- mindestens einer Abstandshalteeinrichtung (21), mit der zumindest bereichsweise ein Abstand (d) zwischen der Plasmaquelle (5) und einer zu behandelnden Oberfläche (47) einhaltbar ist, wobei
- die Plasmaquelle (5) zwei Elektroden (7, 9) umfasst, wobei
- das Gehäuse (3) die beiden Elektroden (7, 9) umfasst, wobei
- die Abstandshalteeinrichtung (21) an dem Gehäuse (3) und/oder der Plasmaquelle (5) angeordnet ist, wobei
- die Abstandshalteeinrichtung (21) derart einstellbar ausgebildet ist, dass
- der Abstand (d) mittels der Abstandshalteeinrichtung (21) derart variierbar ist, dass eine bevorzugte Plasmachemie am Ort der zu behandelnden Oberfläche (47) auswählbar ist, wobei
- mittels der Abstandshalteeinrichtung (21) ein Abstand von 4 bis 30 mm einstellbar ist.

2. Vorrichtung nach Anspruch 1, wobei die Abstandshalteeinrichtung (21) ein Gewinde (23) aufweist das mit einem entsprechenden Gewinde (25) der Vorrichtung (1) kämmt.

3. Vorrichtung nach einem der vorhergehenden Ansprüche, wobei die Abstandshalteeinrichtung (21) mindestens ein Rastelement aufweist, das mit mindestens einem entsprechenden Rastelement der Vorrichtung (1) zusammenwirkt, um den Abstand zu definieren.

4. Vorrichtung nach einem der vorhergehenden Ansprüche, wobei die Vorrichtung, insbesondere die Abstandshalteeinrichtung (21) eine Skala (27) aufweist, anhand derer ein gewünschter Abstand (d) beziehungsweise eine bevorzugte Plasmachemie einstellbar ist.

5. Vorrichtung nach einem der vorhergehenden Ansprüche, wobei die Plasmaquelle (5) eine Oberfläche (13) umfasst, in deren Bereich das Plasma gebildet wird, wobei die Oberfläche (13) vorzugsweise gekrümmt, besonders bevorzugt konvex oder konkav ausgebildet ist.

6. Vorrichtung nach einem der vorhergehenden Ansprüche, wobei eine Applikationseinrichtung (29), durch die mindestens ein Zusatzstoff auf die zu behandelnde Oberfläche (47) aufbringbar ist.

7. Vorrichtung nach Anspruch 6, wobei die Applikationseinrichtung (29) als Spray, Rolleinrichtung oder Stickeinrichtung ausgebildet ist.

8. Vorrichtung nach einem der vorhergehenden Ansprüche, wobei eine Auswahleinrichtung (49), ausgebildet ist zur vorzugsweise automatischen Auswahl einer bevorzugten Plasmachemie.

## Claims

1. Device for plasma treatment of surfaces (47), in particular of skin, comprising
- a housing (3)
- a plasma source (5) associated with the housing (3), and
- at least one spacer device (21), by means of which a distance (d) between the plasma source (5) and a surface (47) being treated can be maintained at least in certain regions, wherein
- the plasma source (5) comprises two electrodes (7, 9), wherein
- the housing (3) comprises the two electrodes (7, 9), wherein
- the spacer device (21) is provided on the housing (3) and/or the plasma source (5), wherein
- the spacer device (21) is configured to be adjustable in such a way that
- the distance (d) can be varied by means of the spacer device (21) in such a way that a preferred plasma chemistry can be selected at the location of the surface (47) being treated, wherein
- a distance of 4 to 30 mm can be set by means of the spacer device (21).

2. Device according to claim 1, wherein the spacer device (21) has a thread (23) which meshes with a corresponding thread (25) of the device (1).

3. Device according to any one of the preceding claims, wherein the spacer device (21) has at least one locking element cooperating with at least one corresponding locking element of the device (1) in order to define the distance.

4. Device according to one of the preceding claims, wherein the device, in particular the spacer device (21), has a scale (27) by means of which a desired distance (d) or a preferred plasma chemistry can be set.

5. Device according to one of the preceding claims, wherein the plasma source (5) comprises a surface (13) in the region of which the plasma is formed, wherein the surface (13) is preferably curved, in particular preferably convex or concave.

6. Device according to one of the preceding claims, comprising an application device (29) by means of which at least one additive can be applied to the surface (47) being treated.

7. Device according to claim 6, wherein the application device (29) is configured as a spray, roller device or stick device.

8. Device according to one of the preceding claims, comprising a selection device (49) which is configured for the preferably automatic selection of a preferred plasma chemistry.

## Revendications

1. Dispositif de traitement par plasma de surfaces (47), en particulier de la peau, avec
- un boîtier (3)
- une source de plasma (5) associée au boîtier (3), et
- au moins un dispositif d'écartement (21) qui permet de maintenir, au moins par zones, une distance (d) entre la source de plasma (5) et une surface (47) à traiter,
- la source de plasma (5) comprenant deux électrodes (7, 9),
- le boîtier (3) comprenant les deux électrodes (7, 9),
- le dispositif d'écartement (21) étant fixé au boîtier (3) et/ou la source de plasma (5),
- le dispositif d'écartement (21) étant conçu de manière à pouvoir être réglé de telle sorte que
- la distance (d) peut être modifiée au moyen du dispositif d'écartement (21) de telle sorte qu'une chimie de plasma préférée peut être sélectionnée à l'endroit de la surface (47) à traiter, dans laquelle
- une distance de 4 à 30 mm peut être réglée au moyen du dispositif d'écartement (21).

2. Dispositif selon la revendication 1, dans lequel le dispositif d'écartement (21) présente un filetage (23) qui s'engrène avec un filetage correspondant (25) du dispositif (1).

3. Dispositif selon l'une des revendications précédentes, dans lequel le dispositif d'écartement (21) présente au moins un élément d'encliquetage qui coopère avec au moins un élément d'encliquetage correspondant du dispositif (1) pour définir l'écartement.

4. Dispositif selon l'une des revendications précédentes, dans lequel le dispositif, en particulier le dispositif d'écartement (21), présente une échelle (27) à l'aide de laquelle une distance souhaitée (d) ou une chimie de plasma préférée peut être réglée.

5. Dispositif selon l'une des revendications précédentes, dans lequel la source de plasma (5) comprend une surface (13) dans la zone de laquelle le plasma est formé, la surface (13) étant conçu de préférence incurvée, de préférence encore convexe ou concave.

6. Dispositif selon l'une des revendications précédentes, comprenant un dispositif d'application (29), par lequel au moins un additif peut être appliqué sur la surface (47) à traiter.

7. Dispositif selon la revendication 6, dans lequel le dispositif d'application (29) est conçu comme un spray, un dispositif à rouleau ou un dispositif de broderie.

8. Dispositif selon l'une des revendications précédentes, comprenant un dispositif de sélection (49) qui est conçu pour la sélection, de préférence automatique, d'une chimie de plasma préférée.
